# EUROPEAN PATENT SPECIFICATION

(11) **EP 3 307 339 B1**
(45) Date of publication and mention of the grant of the patent: **16.12.2020**
(21) Application number: 16729810.8
(22) Date of filing: 31.05.2016
(51) Int. Cl.: A61L 31/10, A61L 31/14

(54) **METHOD FOR BONDING A POLYURETHANE POLYMER TO A SUBSTRATE, IN PARTICULAR FOR THE MANUFACTURING OF STENTS**
VERFAHREN ZUM VERKLEBEN EINES POLYURETHANPOLYMERS MIT EINEM SUBSTRAT, INSBESONDERE ZUR HERSTELLUNG VON STENTS
PROCÉDÉ PERMETTANT DE LIER UN POLYMÈRE DE POLYURÉTHANE À UN SUBSTRAT, EN PARTICULIER DESTINÉ À LA FABRICATION D'ENDOPROTHÈSES

(30) Priority: 15.06.2015 DE 102015109534
(43) Date of publication of application: 18.04.2018
(73) Proprietor: Rheinisch-Westfälische Technische Hochschule (RWTH) Aachen, 52056 Aachen (DE)
(72) Inventor: KIESENDAHL, Nicole, 41844 Wegberg (DE); STEINSEIFER, Ulrich, 4730 Hauset (BE)
(74) Representative: Michalski Hüttermann & Partner Patentanwälte mbB
(86) International application number: PCT/EP2016/062242
(87) International publication number: WO 2016/202574

(56) References cited:
- US-A- 6 017 577

## Description

The present invention relates to a method for bonding a polyurethane polymer to a substrate using a multi-layered adhesion approach. The invention further relates to an article obtainable by the method according to the invention, in particular a biomedical implant such as a heart valve stent.

Stents are widely used for supporting a lumen structure in a patient's body. For example, stents may be used to maintain patency of a coronary artery, other blood vessel or other body lumen.

Commonly, stents are metal, tubular structures. Stents are passed through the body lumen in a collapsed state. At the point of an obstruction or other deployment site in a body lumen, the stent is expanded to an expanded diameter to support the lumen at the deployment site.

In certain designs, stents are open-celled tubes that are expanded by inflatable balloons at the deployment site. This type of stent is often referred to as a "balloon expandable" stent and is often made of a plastically deformable material such as stainless steel. Other stents are so-called "self-expanding" stents. Self-expanding stents do not use balloons to cause the expansion of the stent. An example of a self-expanding stent is a tube (e.g., a coil tube or an open-cell tube) made of an elastically deformable material. Elastically deformable self-expanding stents are typically secured to a stent delivery device under tension in a collapsed state. At the deployment site, the stent is released so that internal tension within the stent causes the steal to self-expand to its enlarged diameter. This type of stent is often made of a "super-elastic" material such as nitinol. Other self-expanding stents are made of so-called shape-memory metals. Such shape-memory stents experience a phase change at the elevated temperature of the human body. The phase change results in expansion from a collapsed state to an enlarged state.

Heart valve replacement is a widely used procedure in the treatment of structural heart disease. For example, heart valve replacement may be indicated when there is a narrowing of the native heart valve, commonly referred to as stenosis, or when the native valve leaks or regurgitates. Prosthetic heart valves used to replace these diseased valves include mechanical and tissue-based valves.

Tissue-based valves include leaflets made from biological tissue such as bovine pericardium or porcine pericardium. For use as valve leaflets, such xenograft tissues typically need to be fixed, usually by glutaraldehyde, and attached to a scaffold, usually by a suture. These processes can be labour intensive and time consuming and, given their manual nature, can be the source of some variability in valve performance.

Polyurethanes based on polycarbonate polyols can be used as biocompatible materials for implantable medical devices such as stents and including heart valve stents. WO 2014/008207 A1 for example discloses a prosthetic heart valve comprising: a base; a plurality of polymeric leaflets, each leaflet having a root portion coupled to the base, each leaflet having an edge portion substantially opposite the root portion and movable relative to the root portion to coapt with a respective edge portion of at least one of the other leaflets of the plurality of leaflets, each leaflet comprising) at least two polymers, and each leaflet having a composition gradient of each of the at least two polymers along at least one portion of the leaflet. Embodiments concern a heart valve wherein each leaflet has a decreasing thickness in a direction extending generally from the root portion to the edge portion and wherein at least one of the at least two polymers is one or more of the following: polycarbonate urethane, poly(dimethylsiloxane urethane) and poly(isobutylene urethane).

The durable bonding of a polymer which has been designed to flex and move during operation onto the frame structure of a stent is a continuing challenge. This is especially the case when the biomedical implant is subject to cyclic mechanical stresses (artificial heart valve) or when the biomedical implant is compressed prior to delivery via a catheter.

Manufacturing processes disclosed in WO 2014/008207 A1 include spray coating processes for forming the prosthetic heart valves by coating the frame and the leaflet form together and a process where a spray coating is applied to the frame and the leaflets are formed separately prior to attaching them to the frame. It is the understanding of the inventors of the present invention that these manufacturing processes lead to an embedding of the frame structure in a polymer base.

Embedding the frame of a biomedical implant in a polymer base to the extent that the actual frame is not discernible any more, as illustrated in FIG. 2 of WO 2014/008207 A1, is disadvantageous because voids in the frame are closed by the polymer. This may have an influence on how the implant is accepted by the body because a larger surface area of a foreign material is presented to the body. In addition, the more polymer base material is present, the more difficult it is to compress the biomedical implant for catheter delivery.

It would be desirable to directly bond a polymeric material to a base material of a biomedical implant. US 4,364,731 teaches a method for producing a dental crown composite comprising the steps of: (a) providing a clean dental crown substrate surface; (b) depositing onto the substrate surface an exogenous coating of inorganic oxide to a desired thickness on the substrate surface to enhance adhesion between the substrate and subsequently applied silane coupling agent; (c) bonding a layer of silane coupling agent to the oxide coating; and (d) applying a polymeric resin to the exposed silane coupling layer. However, there is no indication whether this method would also be suitable for flexible resins outside the field of dental prosthetics.

DE 199 37 864 A1 relates to a workpiece with a substrate of ceramic, metal or polymer, the substrate having a surface which is conditioned to form a stable connection with a polymer and which is provided with a silica layer and, on top of this, with a silane coupling agent. The substrate, the silica layer and the silane coupling agent are sterile and on top of the silane coupling agent, there is a preserving protective layer which is sterile and/or can be sterilized after polymerization.

The Ph.D. thesis "Coating stent materials with polyhedral oligomeric silsesquioxane-poly(carbonateurea) urethane nanocomposites" by Raheleh Bakhshi, University College London, 2009, is concerned with self-expanding/balloon expandable coronary stent design that incorporates a NiTi/stainless steel alloy scaffold with a polyhedral oligomeric silsesquioxane- poly (carbonate-urea) urethane nanocomposite polymer (POSS-PCU) coating. No mention is made of bonding a flexible polymer to the scaffold.

The B.Sc. thesis "Stent Design for a Percutaneous Heart Valve" by Hamresh D. Lutchmun, Worcester Polytechnic Institute 2011 reports that its project is to design a self-expanding Nitinol stent with features to enable attachment to a polyurethane valve, facilitate deployment and repositioning as well as secure placement within the aortic annulus while avoiding obstruction of the aortic nodes. Attachment techniques of the polyurethane valve to a stent include thermoforming and solvent-based techniques.

The publication "Partially Polyurethane-Covered Stent for Cerebral Aneurysm Treatment" by Hussain S. Rangwalalet al., J Biomed Mater Res B Appl Biomater. 2009 May ; 89B(2): 415-429 mentions the use of polycarbonate-based polyurethane as a stent coating but is not concerned with affixing a flexible polymer to a base material where the flexible polymer has a freely movable section as would be the case for prosthetic heart valves.

US 6,017,577 A discloses a process for producing a material bearing thereon a coating of a lubricious, hydrated hydrophilic polyurethane-urea hydrogel, said process comprising the steps of: a) making a surface of a hydrophilic or hydrophilicized hydrophobic polymer substrate reactive by affixing reactive chemical functional groups thereto, at least a portion of which are amine groups; b) coating a reactive polymer substrate surface which results from step (a) with a first coating comprising a hydrophilic polyurethane prepolymer intermediate, containing terminal isocyanate groups, such that a first portion of said terminal isocyanate groups are reacted with and are covalently bonded to said reactive chemical functional groups on said substrate surface, forming covalent urea bonds therewith, resulting in the formation of a tie coat of a polyurethane-urea hydrogel-forming polymer, on said substrate surface, that adheres to said substrate surface, and wherein a second portion of said terminal isocyanate groups of said polyurethane prepolymer intermediate are present in said polyurethane-urea hydrogel-forming polymer such that they remain free to react with other species; and c) coating the tie coat which results from step (b) with a second coating comprising a moisture-containing, hydrogel-forming compound or mixture, which contains isocyanate-reactive functional groups; whereby said isocyanate-reactive functional groups of said hydrogel-forming compound or mixture react with at least a portion of said second portion terminal isocyanate groups of said polyurethane-urea hydrogel-forming polymer; and whereby said moisture of said hydrogel-forming compound or mixture hydrates said polyurethane-urea hydrogel-forming polymer of said tie coat.

The present invention has the object of providing a way to affix a polyurethane polymer to a substrate, in particular in the manufacture of a biomedical implant, wherein the article can be deformed elastically and wherein the polyurethane polymer can withstand dynamic and cyclical loads.

This object has been achieved by a method for bonding a polyurethane polymer to a substrate, the method comprising the steps of:
A) selecting a polyurethane polymer to be bonded to the substrate,
   the polyurethane polymer being obtained by the reaction of a reaction mixture comprising a first isocyanate component and a first isocyanate-reactive component,
   the first isocyanate-reactive component comprising a first polyol,
   the first polyol being a member of a first class of polyols selected from polyether polyols, polyester polyols, polyetherester polyols, polycarbonate polyols, polyether carbonate polyols and polyacrylate polyols;
B) depositing an SiO₂ layer onto the substrate;
C) contacting the SiO₂ layer with a hydroxysilane compound comprising a carbon-carbon double bond and/or with an alkoxysilane compound comprising a carbon-carbon double bond
   and subjecting to conditions suitable to at least partially effect a condensation reaction between Si-OH groups of the SiO₂ layer and the hydroxysilane compound comprising a carbon-carbon double bond and/or the alkoxysilane compound comprising a carbon-carbon double bond,
   thereby forming a first adhesive layer bonded to the SiO₂ layer;
D) contacting the first adhesive layer with a urethane (meth)acrylate compound
   and subjecting to conditions suitable to at least partially effect a reaction between the carbon- carbon double bonds present in the first adhesive layer and the urethane (meth)acrylate compound,
   thereby forming a second adhesive layer bonded to the first adhesive layer;
E) contacting the second adhesive layer with a polyurethane (meth)acrylate compound,
   the polyurethane (meth)acrylate compound being obtained by the reaction of a reaction mixture comprising a second isocyanate component and second isocyanate-reactive component,
   the second isocyanate-reactive component comprising a second polyol,
   the second polyol being a member of the first class of polyols as mentioned in step A),
   the polyurethane (meth)acrylate compound furthermore comprising (meth)acrylate groups
   and subjecting to conditions suitable to at least partially effect a reaction between the (meth)acrylate groups and/or carbon radicals present in the second adhesive layer and the polyurethane (meth)acrylate compound,
   thereby forming a third adhesive layer bonded to the second adhesive layer;
F) contacting the third adhesive layer with the polyurethane polymer selected in step A),
   the polyurethane polymer being in a molten and/or dissolved state;
G) solidifying the polyurethane polymer of step F) which is in contact with the third adhesive layer by
   cooling the polyurethane polymer below its melting point in the case that it has been employed in a molten state in step F);
   and/or
   evaporating the solvent in which the polyurethane polymer has been dissolved in the case that it has been employed in a dissolved state in step F);
   thereby obtaining a polyurethane polymer layer;
   thereby obtaining an article comprising a polyurethane polymer bonded to a substrate.

While the method according to the invention has been developed with respect to the manufacture of biomedical implants, it is by no means limited to this technical field.

The substrate to which the polyurethane polymer is to be bonded may be a metal or a ceramic substrate. Preferably, it is a biologically compatible substrate suitable for implantation into a patient.

According to step A) of the method, a polyurethane polymer which is to be bonded to the substrate is selected. This step does not strictly need to be the first step of the method according to the invention as long as it is performed before step F).

The polyurethane polymer may, for example, form a heart valve leaflet or a fleece surrounding a stent. It is obtained by the reaction of a reaction mixture comprising an isocyanate component which has been designated as "first isocyanate component" and an isocyanate-reactive component which has been designated as "first isocyanate-reactive component".

Examples for an isocyanate of the first isocyanate component include aliphatic diisocyanates such as tetramethylene diisocyanate, hexamethylene diisocyanate (1,6-diisocyanatohexane), octamethylene diisocyanate, decamethylene diisocyanate, dodecamethylene diisocyanate, tetradecamethylene diisocyanate, trimethylhexane diisocyanate or tetramethylhexane diisocyanate, cycloaliphatic diisocyanates such as 1,4-, 1,3- or 1,2-diisocyanatocyclohexane, 4,4'-di(isocyanatocyclohexyl)methane, 1-isocyanato-3,3,5 -trimethyl-5 -isocyanatomethylcyclohexane (isophorone diisocyanate) or 2,4-, or 2,6-diisocyanato-1-methylcyclohexane and also aromatic diisocyanates such as 2,4- or 2,6-tolylene diisocyanate, tetramethylxylylene diisocyanate, p-xylylene diisocyanate, 2,4'- or 4,4'-diisocyanatodiphenylmethane, 1,3- or 1,4-phenylene diisocyanate, 1-chloro-2,4-phenylene diisocyanate, 1,5-naphthylene diisocyanate, diphenylene 4,4'-diisocyanate, 4,4'-diisocyanato-3,3'-dimethyldiphenyl, 3-methyldiphenylmethane 4,4'-diisocyanate or diphenyl ether 4,4'-diisocyanate. Mixtures of the diisocyanates mentioned can also be present.

Aliphatic isocyanates are preferred.

Suitable customary more highly functionalized polyisocyanates are, for example, triisocyanates such as 2,4,6-triisocyanatotoluene or 2,4,4'-triisocyanatodiphenyl ether or the mixtures of di-, tri- and higher polyisocyanates which are obtained by phosgenation of appropriate aniline/formaldehyde condensates and represent polyphenyl polyisocyanates having methylene bridges.

Also suitable are customary aliphatic more highly functionalized polyisocyanates of the following groups:
(a) Isocyanurate-containing polyisocyanates of aliphatic and/or cycloaliphatic diisocyanates. Particularly preferred in this context are the corresponding isocyanato isocyanurates based on hexamethylene diisocyanate and isophorone diisocyanate. The isocyanurates are, in particular, simple trisisocyanatoalkyl or tris-isocyanatocycloalkyl isocyanurates which are cyclic trimers of the diisocyanates, or mixtures with their higher homologs containing more than one isocyanurate ring. The isocyanato iscyanurates in general have an NCO content of from 10 to 30% by weight, in particular 15 to 25% by weight, and a mean NCO functionality of from 2,6 to 4,5.
(b) Uretdione diisocyanates having aliphatically and/or cycloaliphatically bonded isocyanate groups, preferably derived from hexamethylene diisocyanate or isophorone diisocyanate. Uretdione diisocyanates are cyclic dimerization products of diisocyanates.
(c) Biuret-containing polyisocyanates having aliphatically bonded isocyanate groups, in particular tris(6-isocyanatohexyl)biuret or its mixtures with its higher homologs. These biuret-containing polyisocyanates in general have an NCO content of from 10 to 30 % by weight, in particular from 18 to 25% by weight, and a mean NCO functionality of from 3 to 4,5.
(d) Urethane- and/or allophanate-containing polyisocyanates having aliphatically or cycloaliphatically bonded isocyanate groups, as can be obtained, for example, by reaction of excess amounts of hexamethylene diisocyanate or of isophorone diisocyanate with simple polyhydric alcohols such as trimethylolpropane, glycerol, 1,2-dihydroxypropane or mixtures thereof. These urethane- and/or allophanate-containing polyisocyanates in general have an NCO content of from 12 to 20% by weight and a mean NCO functionality of from 2.5 to 3.
(e) Oxadiazinetrione-containing polyisocyanates, preferably derived from hexamethylene diisocyanate or isophorone diisocyanate. Such oxadiazinetrione-containing polyisocyanates can be prepared from diisocyanate and carbon dioxide.
(f) Uretonimine-modified polyisocyanates.

The first isocyanate-reactive component comprises a polyol which has been designated as "first polyol". Depending on the desired polyurethane the isocyanate-reactive component may also comprise other polyols, chain extenders, auxiliary substances and the like. It is preferred that the first polyol is the major constituent (by weight) of the isocyanate-reactive component.

The first polyol is a member of a class of polyols designated as "first class of polyols". The class of polyols is selected from polyether polyols, polyester polyols, polyetherester polyols, polycarbonate polyols, polyether carbonate polyols and polyacrylate polyols. By choosing the polyol from the class of polyether polyols, it is possible to refer to the polyurethane polymer as a "polyether polyol based polyurethane". The same is mutatis mutandis the case for the other classes of polyols, e.g. a polycarbonate polyol based polyurethane.

It is of course also possible that more than one polyols of one class are selected, for instance two distinct polyether polyols or two distinct polycarbonate polyols. Then it is preferred that the combined weight of the polyols of the first class of polyols constitutes the major constituent (by weight) of the first isocyanate-reactive component.

The reasons for defining the polyurethane to be selected in this way will become apparent when discussing step E) of the method according to the invention.

Step B) of the method involves depositing an SiO₂ layer onto the substrate. As it is known, the surface of an SiO₂ layer also has Si-OH groups which are available for further functionalization of the surface. This may be undertaken via vapour deposition such as chemical vapour deposition (CVD) or physical vapour deposition (PVD). It is preferred that the SiO₂ layer is thin, for example having a thickness of ≥ 50 nm to ≤ 100 nm, more preferred ≥ 60 nm to ≤ 90 nm.

If the substrate already comprises SiO₂ on its surface, as would be the case for silica or a silica-containing ceramic, then step B) is considered to already having taken place.

It is possible to repeat step B) a number of times, for example twice, three times or four times, before continuing with the next step.

In the next step of the method according to the invention, step C), the SiO₂ layer is contacted with a hydroxysilane compound comprising a carbon-carbon double bond and/or with an alkoxysilane compound comprising a carbon-carbon double bond. These difunctional compounds can react with other molecules via their C=C double bonds and with Si-OH groups via their hydroxysilane or alkoxysilane groups.

To this end, after the SiO₂ layer of the substrate has been contacted with the difunctional compound, the structure is subjected to silanol condensation conditions. These may include heating to a temperature of ≥ 50 °C to ≤ 150 °C, optionally in the presence of water vapor. This condensation reaction links the formerly difunctional molecule to the SiO₂ layer. A monomolecular layer is sufficient for the purposes of the present invention. This may be achieved by rinsing the surface with an inert solvent after the condensation reaction or by employing a dilute solution of the compound, followed by evaporation of the solvent. Thicker layers of the difunctional molecule are not detrimental, however, since they will also react in subsequent photochemical reactions.

As a result of step C), a first adhesive layer bonded to the SiO₂ layer is formed. Owing to the fact that a monomolecular layer may be sufficient, the thickness of the first adhesive layer may be very low.

It is possible to repeat step C) a number of times, for example twice, three times or four times, before continuing with the next step.

Step D) of the method according to the invention concerns the next adhesive layer. The first adhesive layer of step C) is contacted with a urethane (meth)acrylate compound. This may of course also be a polyurethane (meth)acrylate compound. Such compounds are commonly found in commercial UV and/or visible light curing adhesives. Hence, this urethane (meth)acrylate compound may be viewed as an adhesive. An example is the acrylated urethane adhesive Loctite® 3301. In general, urethane (meth)acrylate compounds may be manufactured by including a hydroxyalkyl (meth)acrylate such as HEMA (hydroxyethyl methacrylate) into a urethane reaction mixture.

Then, the structure is subjected to conditions suitable to at least partially effect a reaction between the C=C double bonds present in the first adhesive layer (derived from the difunctional compound as discussed in connection with step C)) and the urethane (meth)acrylate compound, in particular with the (meth)acrylate groups of this compound. Conditions may be thermal treatment and/or irradiation with light. The presence of a radical initiator may accelerate the reaction.

While it is not detrimental to fully cure this intermediate assembly during step D), it is advantageous to leave some C=C double bonds and/or (meth)acrylate groups intact. This will lead to a looser network in this layer, allowing polymers which are applied in subsequent steps to interpenetrate and to create stronger bonds between the layers.

The process parameters for a partial cure may be derived from experimentation or from consulting product data sheets to determine, for example, the UV radiation intensity after which, for example the viscosity of the substance reaches 25%, 50% or 75% of its final viscosity as a cured product. Then the process parameters can be chosen such as to not fully cure the intermediate assembly.

As a result of step D), a second adhesive layer bonded to the first adhesive layer is formed. The total thickness of the SiO₂ layer, the first adhesive layer and the second adhesive layer may be in a range of ≥ 5 µm to ≤ 10 µm.

It is possible to repeat step E) a number of times, for example twice, three times or four times, before continuing with the next step.

In step E) of the method according to the invention the second adhesive layer is contacted with another polyurethane (meth)acrylate compound. This compound is obtained by the reaction of a reaction mixture comprising an isocyanate component designated as "second isocyanate component" and an isocyanate-reactive component designated as "second isocyanate-reactive component".

Examples for an isocyanate of the second isocyanate component include those outlined in connection with the first isocyanate component. They will not be repeated here for the sake of brevity. The second isocyanate component may be the same as the first isocyanate component or different from the first isocyanate component.

The second isocyanate-reactive component comprises a polyol which has been designated as "second polyol". Depending on the desired polyurethane the isocyanate-reactive component may also comprise other polyols, chain extenders, auxiliary substances and the like. It is preferred that the second polyol is the major constituent (by weight) of the isocyanate-reactive component.

The second polyol is a member of the same class of polyols as the first polyol (cf. step A)). Hence, if the polymer selected in step A) is a polyether polyol based polyurethane, a polyether polyol based polyurethane (meth)acrylate compound is employed in step E). The same is mutatis mutandis the case for the other classes of polyols, e.g. a polycarbonate polyol based polyurethane (meth) acrylate compound.

It is of course also possible that more than one polyols of one class are selected, for instance two distinct polyether polyols or two distinct polycarbonate polyols. Then it is preferred that the combined weight of the polyols of the first class of polyols constitutes the major constituent (by weight) of the second isocyanate-reactive component.

It is also provided that this polyurethane compound furthermore comprises (meth)acrylate groups. In general, such (meth)acrylate compounds may be manufactured by including a hydroxyalkyl (meth)acrylate such as HEMA (hydroxyethyl methacrylate) into a polyurethane reaction mixture.

The selection instructions of steps A) and E) have the effect that chemically similar polyurethanes are employed in the process without necessitating identity of the polyurethanes. This increases the bonding between the layers. By way of example, a polycarbonate polyol based polyurethane could be selected in step A) and a polycarbonate polyol based polyurethane methacrylate compound could be selected in step E), wherein the polyols used in the synthesis of these two polymers could differ (e. g. with respect to Mₙ, M_{w}, functionality, etc.) but still ensuring chemical compatibility due to the fact that both polyurethanes are made from polycarbonate polyols.

According to step E) the resulting structure is subjected to conditions suitable to at least partially effect a reaction between the (meth)acrylate groups and/or carbon radicals present in the second adhesive layer and the polyurethane (meth)acrylate compound. Conditions may be thermal treatment and/or irradiation with light. The presence of a radical initiator may accelerate the reaction.

It is advantageous to fully cure the assembly in step E). This creates firm bonds between the layers, including the reaction of previously unreacted C=C double bonds of the first adhesive layer.

As a result of step E), a third adhesive layer bonded to the second adhesive layer is formed. The thickness of such a layer may be in a range of ≥ 1 µm to ≤ 100 µm, more preferred ≥ 5 µm to ≤ 50 µm.

It is possible to repeat step E) a number of times, for example twice, three times or four times, before continuing with the next step.

In step F) the third adhesive layer is contacted with the polymer selected in step A). For better adhesion, it is provided that the polymer is in a molten and/or dissolved state. Hence, the polyurethane inherently has the property that it is thermoplastic or at least soluble in solvent. A firm bond between the polymer and the third adhesive layer is achieved by their chemical similarity. It is possible to repeat this step a number of times, for example twice, three times or four times, before continuing with the next step.

Step G) concerns the solidification of the polymer applied in step F). The thickness of the thus obtained polyurethane polymer layer is not limited in principle. For example, the edge of a sheet of polyurethane polymer may be thermoformed onto the third adhesive layer.

It is also possible to conduct steps F) and G) in such a way that a solid (undissolved, below its melting point) polyurethane polymer as selected in step A) is brought into contact with the third adhesive layer and a melt and/or solution of this polymer is applied to the contact area. After cooling/evaporating the bond is formed.

The present invention will be further described in connection with further aspects and embodiments. They may be combined freely unless the context clearly indicates otherwise.

In one embodiment of the method according to the invention the material of the substrate comprises nitinol (nickel titanium alloy), aluminium, steel and/or titanium. Nitinol is preferred due to its shape-memory properties.

In another embodiment of the method according to the invention the first polyol of the polyurethane polymer of step A) is a polycarbonate polyol. Suitable polycarbonate polyols include the products obtained from the reaction of diols such as propanediol-(1,3), butanediol-(1,4) and/or hexanediol-(1,6), diethylene glycol, bisphenol-A, triethylene glycol or tetraethylene glycol with diarylcarbonates, e.g. diphenylcarbonate, dimethylcarbonate, diethyleneglycol carbonate or phosgene.

Especially preferred with respect to step A) are aliphatic thermoplastic polyurethanes (TPU) where the first polyol is a polycarbonate polyol (also designated as polycarbonate urethanes, PCU). Examples for suitable material properties are given in the table below.

| **Property** | **ASTM test** | **PCU 1** | **PCU 2** | **PCU 3** | **PCU 4** | **PCU 5** |
|---|---|---|---|---|---|---|
| Shore hardness | D2240 | 70A | 80A | 90A | 50D | 69D |
| Specific gravity | D792 | 1.15 | 1.15 | 1.15 | 1.15 | 1.15 |
| Ultimate tensile (psi) | D412 | 7200 | 8300 | 9600 | 9700 | 9600 |
| Ultimate elongation (%) | D412 | 600 | 425 | 400 | 325 | 325 |
| Tensile (psi)... | D412 | | | | | |
| ... at 100% elongation | | 350 | 600 | 1050 | 1750 | 3500 |
| ... at 200% elongation | | 500 | 1100 | 2250 | 3100 | 5400 |
| ... at 300% elongation | | 2000 | 4000 | 6400 | 6800 | 9200 |
| Flexural modulus (psi) | D790 | 1600 | 1470 | 6400 | 19300 | 134000 |
| Vicat temp. (°C) | D1525 | 46 | 58 | 62 | 51 | 63 |
| Mould shrinkage (in/in) (1" ^{∗} 25" ^{∗} 6" bar) | D955 | 0.010 | 0.010 | 0.010 | 0.008 | 0.0008 |

In another embodiment of the method according to the invention in step B) the SiO₂ layer is deposited by physical vapour deposition.

In another embodiment of the method according to the invention in step C) the contacting of the SiO₂ layer is undertaken with an alkoxysilane compound comprising a (meth)acrylate group. Preferably, the compound is selected from the group of (3-acryloxypropyl)trimethoxysilane, methacryloxypropyltrimethoxysilane, N-(3-acryloxy-2-hydroxypropyl)-3-aminopropyltriethoxysilane, O-(methacryloxyethyl)-n-(triethoxysilylpropyl)urethane, N-(3-methacryloxy-2-hydroxypropyl)-3-aminopropyltriethoxysilane, methacryloxypropyltriethoxysilane, methacryloxymethyl-triethoxysilane, methacryloxymethyltrimethoxysilane, (3-acryloxypropyl)methyldimethoxysilane, (methacryloxymethyl)methyldiethoxysilane, (methacryloxymethyl)methyldimethoxysilane, methacryloxypropylmethyldiethoxysilane, methacryloxypropylmethyldimethoxysilane, methacryl-oxypropyldimethylethoxysilane and/or methacryloxypropyldimethylmethoxysilane.

In another embodiment of the method according to the invention in step D) the conditions are: irradiating with ultraviolet light in the presence of a UV initiator at an intensity of ≥ 5 mW/cm² to ≤ 50 mW/cm² for a duration of ≥ 1 minute to ≤ 10 minutes. Examples for UV initiators include acetophenone, anisoin, anthraquinone, anthraquinone-2-sulfonic acid, sodium salt monohydrate, (benzene) tricarbonylchromium, benzil, benzoin, benzoin ethyl ether, benzoin isobutyl ether, benzoin methyl ether, benzophenone, benzophenone/1-hydroxycyclohexyl phenyl ketone (50/50 blend), 3,3',4,4'-benzophenonetetracarboxylic dianhydride, 4-benzoylbiphenyl, 2-benzyl-2-(dimethylamino)-4'- morpholinobutyrophenone, 4,4'-bis(diethylamino)benzophenone, 4,4'-bis(dimethylamino)benzophenone, camphorquinone, 2-chlorothioxanthen-9-one, (cumene)cyclopentadienyliron(ii) hexafluorophosphate, dibenzosuberenone, 2,2-diethoxyacetophenone, 4,4'-dihydroxybenzophenone, 2,2-dimethoxy-2-phenylacetophenone, 4-(dimethylamino)benzophenone, 4,4'-dimethylbenzil, 2,5-dimethylbenzophenone, 3,4-dimethylbenzophenone, diphenyl(2,4,6 trimethylbenzoyl)phosphine oxide/2-hydroxy-2-methylpropiophenone (50/50 blend), 4'-ethoxyacetophenone, ethylanthraquinone, ferrocene, 3'-hydroxyacetophenone, 4'-hydroxyacetophenone, 3-hydroxybenzophenone, 4-hydroxybenzophenone, 1-hydroxycyclohexyl phenyl ketone, 2-hydroxy-2-methylpropiophenone, 2-methylbenzophenone, 3-methylbenzophenone, methybenzoylformate, 2-methyl-4'-(methylthio)-2- morpholinopropiophenone, phenanthrenequinone, 4'-phenoxyacetophenone, thioxanthen-9-one, triarylsulfonium hexafluoroantimonate salts and/or triarylsulfonium hexafluorophosphate salts.

UV irradiation may be performed using a Hg vapour lamp or an ultraviolet LED. Preferred maximum wavelengths for UV irradiation are 254 nm and/or 365 nm. UV-A light with a wavelength from 315 to 380 nm may be used. Preferred parameters are ≥ 10 mW/cm² to ≤ 30 mW/cm² for a duration of ≥ 2 minutes to ≤ 9 minutes.

In case the determination of a process parameter expressed as mW/cm² may not be practical due to the small dimensions of the surface involved, using a UV lamp with a power output between 30 and 50 W may suffice, especially with objects having the dimensions of biomedical implants.

In another embodiment of the method according to the invention in step E) the conditions are: irradiating with ultraviolet light in the presence of a UV initiator at an intensity of ≥ 5 mW/cm² to ≤ 100 mW/cm² for a duration of ≥ 1 minute to ≤ 10 minutes. Examples for UV initiators include acetophenone, anisoin, anthraquinone, anthraquinone-2-sulfonic acid, sodium salt monohydrate, (benzene) tricarbonylchromium, benzil, benzoin, benzoin ethyl ether, benzoin isobutyl ether, benzoin methyl ether, benzophenone, benzophenone/1-hydroxycyclohexyl phenyl ketone (50/50 blend), 3,3',4,4'-benzophenonetetracarboxylic dianhydride, 4-benzoylbiphenyl, 2-benzyl-2-(dimethylamino)-4'- morpholinobutyrophenone, 4,4'-bis(diethylamino)benzophenone, 4,4'-bis(dimethylamino)benzophenone, camphorquinone, 2-chlorothioxanthen-9-one, (cumene)cyclopentadienyliron(ii) hexafluorophosphate, dibenzosuberenone, 2,2-diethoxyacetophenone, 4,4'-dihydroxybenzophenone, 2,2-dimethoxy-2-phenylacetophenone, 4-(dimethylamino)benzophenone, 4,4'-dimethylbenzil, 2,5-dimethylbenzophenone, 3,4-dimethylbenzophenone, diphenyl(2,4,6 trimethylbenzoyl)phosphine oxide/2-hydroxy-2-methylpropiophenone (50/50 blend), 4'-ethoxyacetophenone, ethylanthraquinone, ferrocene, 3'-hydroxyacetophenone, 4'-hydroxyacetophenone, 3-hydroxybenzophenone, 4-hydroxybenzophenone, 1-hydroxycyclohexyl phenyl ketone, 2-hydroxy-2-methylpropiophenone, 2-methylbenzophenone, 3-methylbenzophenone, methybenzoylformate, 2-methyl-4'-(methylthio)-2- morpholinopropiophenone, phenanthrenequinone, 4'-phenoxyacetophenone, thioxanthen-9-one, triarylsulfonium hexafluoroantimonate salts and/or triarylsulfonium hexafluorophosphate salts. Preferred is a mixture of benzophenone and 1-hydroxycyclohexyl phenyl ketone as the UV initiator.

UV irradiation may be performed using a Hg vapour lamp or an ultraviolet LED. Preferred maximum wavelengths for UV irradiation are 254 nm and/or 365 nm. UV light with a wavelength from 320 to 500 nm may be used Preferred parameters are ≥ 20 mW/cm² to ≤ 50 mW/cm² for a duration of ≥ 3 minutes to ≤ 9 minutes.

In another embodiment of the method according to the invention in step F) the third adhesive layer is contacted with a solution of the polyurethane polymer selected in step A) and with solid polyurethane selected in step A). The solution of the polymer will also superficially dissolve or swell the surface of the solid polymer. After evaporation of the solvent, the solid polyurethane is bond to the third adhesive layer via the previously dissolved polyurethane.

In another embodiment of the method according to the invention in step F) the third adhesive layer is contacted with a solution of the polyurethane polymer selected in step A) by spraying, through a nozzle moving relative to the third adhesive layer, a solution of the polyurethane polymer selected in step A) onto the third adhesive layer. This allows the fabrication of polyurethane fleeces bond to the third adhesive layer. If a fleece is desired, the evaporation characteristics of the solvent should be chosen such as to deposit an at least partially solidified polyurethane fibre onto the third adhesive layer.

In another embodiment of the method according to the invention in step E) the second adhesive layer is contacted with a mixture of the polyurethane (meth)acrylate compound and a solution of the polyurethane polymer selected in step A). This may occur instead of the contacting of the second adhesive layer with a polyurethane (meth)acrylate compound according to step E) or as an additional step within step E). If it conducted as an additional step, curing of the polymers can occur before and after the additional step. Physically mixing these two materials in solution leads to a greater chemical similarity of the surface of the layer and thus to a stronger bond to the polymer as selected in step A).

The present invention is furthermore directed towards an article comprising a polyurethane polymer layer bonded to a substrate, wherein the polyurethane polymer layer is bonded to the substrate by an adhesive layer assembly comprising:
an SiO₂ layer deposited onto the substrate;
a first adhesive layer bonded to the SiO₂ layer;
a second adhesive layer bonded to the first adhesive layer;
a third adhesive layer bonded to the second adhesive layer and bonded to the polyurethane;
the adhesive layer assembly obtainable (or obtained) by a method according to the present invention.

Reference has already been made to the details of the substrate (preferably, nitinol), the polyurethane of the polyurethane layer and the adhesive layers with respect to the method according to the invention. They are applicable as well for the article according to the invention and will not be repeated here for the sake of avoiding repetition.

In one embodiment of the article according to the invention the polyurethane polymer of the polyurethane polymer layer is obtained by the reaction of a reaction mixture comprising a first isocyanate component and a first isocyanate-reactive component, the first isocyanate-reactive component comprising a first polyol, the first polyol being a member of the class of polycarbonate polyols. Suitable polycarbonate polyols and the polyurethanes derived from them have been described in the context of the method according to the invention.

In another embodiment of the article according to the invention the polyurethane layer bridges opposing sections of the substrate.

In another embodiment of the article according to the invention the polyurethane layer is disposed as a fleece.

In another embodiment of the article according to the invention the article is a biomedical implant. Preferably, the article is a stent such as a heart valve stent or a pulmonary stent.

The present invention will be further described with reference to the following figures and examples without wishing to be limited by them.
- FIG. 1a-1e: show various steps of the method according to the invention
- FIG. 2-4: show biomedical implants or their parts according to the invention

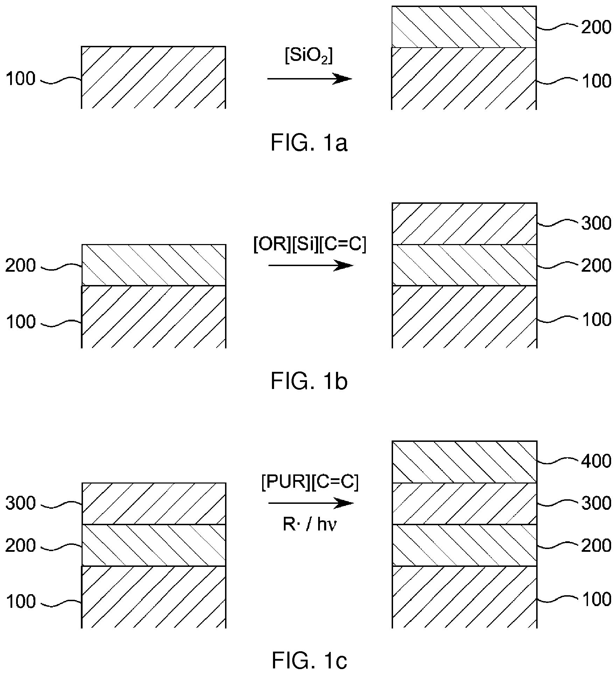

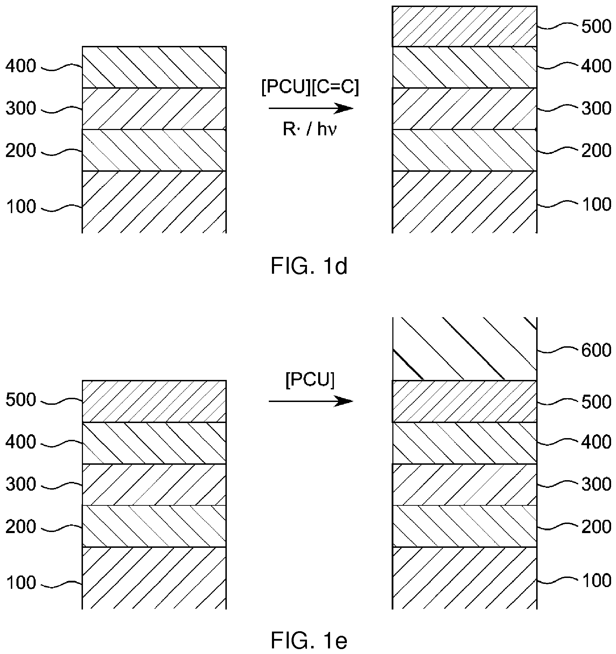

In FIG. 1a it is depicted that onto a substrate 100, in particular a nitinol wire, a silicon dioxide layer 200) is deposited. Preferably this is undertaken using physical vapour deposition. The step depicted in this figure corresponds to step B) of the method according to the invention.

The next step, step C), is shown in FIG. 1b. The silicon dioxide layer 200 is contacted with an alkoxysilane compound comprising a carbon-carbon double bond [OR][Si][C=C]. Preferably it is methacryloxypropyltrimethoxysilane which is used as the alkoxysilane compound. After contacting and establishment of favourable conditions the alkoxysilane undergoes a condensation reaction with Si-OH groups present on the surface of the silicon dioxide layer 200 while the corresponding alcohol (in the preferred case, methanol) is released. The first adhesive layer 300 which is thereby formed comprises C=C double bonds, preferably in the form of methacrylate groups, which are available for further functionalization.

FIG. 1c is concerned with step D) of the method. The first adhesive layer 300 is contacted with a urethane (meth)acrylate compound [PUR][C=C]. Preferably, the commercially available acrylated urethane adhesive Loctite® 3301 is used which also contains a suitable photo initiator. An excess of (meth)acrylate groups from the adhesive with respect to the methacrylate groups in the first adhesive layer 300 may be used. Photopolymerization using UV-A light with a wavelength between 315 nm and 380 nm chemically bonds the adhesive to the first adhesive layer 300 under formation of a second adhesive layer 400.

It should be appreciated that after the photopolymerization as depicted in FIG. 1c radicals on the reaction product of the urethane acrylate adhesive and the reactive groups of the second first adhesive layer 300 may be present. This is desirable as it leads to a more efficient chemical bonding in the subsequent step E).

As shown in FIG. Id, corresponding to step E) of the method according to the invention, the second adhesive layer 400 is contacted with a polycarbonate-based polyurethane (meth)acrylate compound [PCU] [C=C] and a photopolymerization (using an initiator and UV irradiation) is conducted. The (meth)acrylate groups of [PCU][C=C] react with radicals and (meth)acrylate groups of the second adhesive layer 400 and with themselves to give a third adhesive layer 500 bonded to the second adhesive layer 400.

Up to this step in the method according to the invention a structure of firmly bonded layers has been established on the nitinol wire substrate 100. Due to chemical similarities a gradual transition from a nitinol wire surface to a surface having the characteristics of a polycarbonate polyol-based polyurethane has been achieved. Hence, the surface is prepared to accept the actual polyurethane to be bonded as selected in step A) without the necessity of forming covalent bonds.

According to FIG. 1e, broadly corresponding to steps F) and G) of the method according to the invention, a solution of a polycarbonate polyol-based polyurethane [PCU] is brought into contact with the third adhesive layer. A suitable solvent is chloroform. After evaporation of the chloroform, the polyurethane polymer layer 600 is obtained.

### Examples

In the examples, the abbreviations have the following meaning:
- PCU:: polycarbonate polyol-based polyurethane
- Loctite® 3301:: UV adhesive; acrylated urethane
- Carbothane® 125:: acrylated PCU based on Carbothane® PC-3575A
- Carbothane® PC-3585A:: PCU (abbreviated to Carbothane® 3585)
- Carbothane® PC-3575A:: PCU (abbreviated to Carbothane® 3575)
- Adiamat® XS:: PCU; comparable to Carbothane® PC-3575A
- DMAc:: N,N-dimethyl acetamide
- MEMO:: methacryloxypropyltrimethoxy silane

Carbothane® 3585 and 3575 have the following properties:

| **Property** | **ASTM test** | **3575** | **3585** |
|---|---|---|---|
| Shore hardness | D2240 | 70A | 80A |
| Specific gravity | D792 | 1.15 | 1.15 |
| Ultimate tensile (psi) | D412 | 7200 | 8300 |
| Ultimate elongation (%) | D412 | 600 | 425 |
| Tensile (psi)... | D412 | | |
| ... at 100% elongation | | 350 | 600 |
| ... at 200% elongation | | 500 | 1100 |
| ... at 300% elongation | | 2000 | 4000 |
| Flexural modulus (psi) | D790 | 1600 | 1470 |
| Vicat temp. (°C) | D1525 | 46 | 58 |
| Mould shrinkage (in/in) (1" ^{∗} 25" ^{∗} 6" bar) | D955 | 0.010 | 0.010 |

### Example 1: tension test (comparative example)

The surface of a nitinol plate was roughened by sand blasting and cleaned using isopropanol and ultrasound. Four layers of a PCU solution (Adiamat® XS, 15 weight-% in DMAc) were subsequently applied using a spatula. The waiting time between applications was 24 hours in order to allow the solvent to evaporate.

A strip of PCU was prepared by spin casting a solution of 10 weight-% Carbothane® 3585 in chloroform and evaporation of the solvent. This strip was bonded to the PCU layers on the metal plate using a 10 weight-% solution of Carbothane® 3585 by applying pressure for 1 minute and allowing the solvent to evaporate for 24 hours. The contact area between the PCU layers on the metal plate and the PCU strip was 10 mm x 10 mm.

A 90° peel test was performed on several samples. Peeling resistance values between 1.7 and 2.3 N/mm were obtained.

### Example 2: tension test

The PCU material Carbothane® 3585 was selected as the polymer to be bonded to a nitinol plate (step A) of the method according to the invention).

The surface of a nitinol plate was cleaned using isopropanol and blasted with corundum particles (110 µm). The cleaned surface was then provided with a silica layer using physical vapour deposition (step B) of the method according to the invention).

The SiO₂ layer was then contacted with methacryloxypropyltrimethoxy silane (MEMO). Condensation of MEMO with Si-OH groups of the SiO₂ layer occurred spontaneously (step C) of the method according to the invention).

Without undue delay, the acrylated urethane adhesive Loctite® 3301 was applied on top of the surface formed by MEMO or its reaction products, respectively. This assembly was irradiated with UV light, thereby fulfilling step D) of the method according to the invention.

A solution of 10 weight-% of Carbothane® 125 in chloroform with 4 weight-% of benzophenone and 2 weight-% of hydroxycyclohexylphenyl ketone (based on the weight of Carbothane® 125) was applied onto the cured layer of the previous step and subjected to UV light (wavelength 320 to 500 nm; 3 minutes; 3 to 4 cm distance from light source). This concludes step E) of the method according to the invention. Visual inspection of the cured assembly indicated a glossy finish as opposed to a matte appearance before UV irradiation.

Four layers of a PCU solution (Adiamat® XS, 15 weight-% in DMAc) were subsequently applied using a spatula. The waiting time between applications was 24 hours in order to allow the solvent to evaporate. In summary, step F) of the method according to the invention has been fulfilled.

A strip of PCU was prepared by spin casting a solution of 10 weight-% Carbothane® 3585 in chloroform and evaporation of the solvent. This strip was bonded to the PCU layers on the metal plate using a 10 weight-% solution of Carbothane® 3585 by applying pressure for 1 minute and allowing the solvent to evaporate for 24 hours. The contact area between the PCU layers on the metal plate and the PCU strip was 10 mm x 10 mm. This concludes step F) of the method according to the invention and in particular the embodiment wherein in step F) the third adhesive layer is contacted with a solution of the polyurethane polymer selected in step A) and with solid polyurethane selected in step A). Furthermore, step G) has been fulfilled by evaporating the solvent.

A 90° peel test was performed on several samples. Peeling resistance values between 3.4 and 4.0 N/mm were obtained.

### Example 3: stent frame

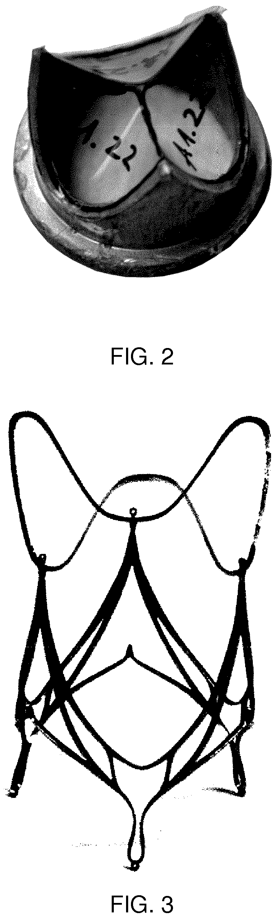

The edges of an eloxated aluminium stent frame were sanded with sanding paper and then treated according to the protocol as outlined in example 2. Three leaflets of Carbothane® 3585 were bonded to the stent frame to form a model heart valve. The finished assembly is depicted in FIG. 2. The transparent leaflets bear the handwritten marking "A1.22" in this photography.

This stent frame was tested in a continuous heart valve testing facility at 37 °C in an aqueous medium under cyclic load. In two samples, no failure was detected after ca. 35 million cycles. Reference samples were also prepared according to the protocol of example 1. These stent frames showed failure after ca. 1.4 million cycles and ca. 32 million cycles, respectively.

### Example 4: heart valve stent

In this example, a collapsible heart valve stent made of nitinol as depicted in FIG. 3 was used. The areas to be coated were washed with isopropanol and blasted with corundum (50 µm). PVD of a SiO₂ layer, silane exposure and the application of the UV adhesive (step D) of the method according to the invention) were undertaken as outlined in example 1. Liquid chemicals were applied to the stent using a brush.

For step E) of the method according to the invention the stent was dipped into a solution of 10 weight-% of Carbothane® 125 in chloroform with 4 weight-% of benzophenone and 2 weight-% of hydroxycyclohexylphenyl ketone (based on the weight of Carbothane® 125) three times and extracted slowly to prevent the formation of polymer films over window sections of the stent. After the third dipping the stent was rotated horizontally at ca. 250 rpm and subjected to UV light (wavelength 320 nm to 500 nm; 3 minutes; 3 to 4 cm distance from light source). Then the stent was dipped into the Carbothane® 125 solution once again, extracted slowly and cured by UV light under rotation. Macroscopic and microscopic inspection showed the coated areas of the stent to have a glossy surface appearance.

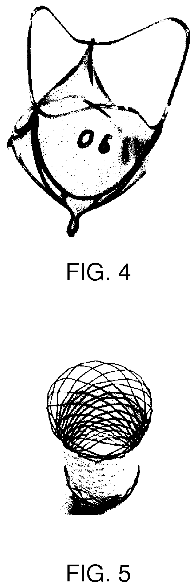

After a waiting period of 24 hours in order to allow the solvent to evaporate, a thermoformed PCU tube (Carbothane® 3585) in ventricular shape was bonded with the aid of a 10 weight-% solution of this PCU in chloroform to the exterior of the stent, giving rise to the finished product after evaporation of the solvent as depicted in FIG. 4. The manual marking of "06" is on the PCU material forming a ventricle.

The durability of several specimens thus obtained was also tested in a continuous heart valve testing facility at 37 °C in an aqueous medium under dynamic load (500 to 800 beats per minute, 100 mm Hg pressure). Mechanical failure occurred after ca. 80 million and ca. 112 million cycles, respectively. The mode of failure was a fracture of the nitinol stent itself and a tearing of the bulk PCU material. The bond between the PCU material and the stent substrate remained intact.

### Example 5: heart valve stent (modified procedure)

In order to further improve the adhesion of the bulk PCU material, a modified procedure was tested. This modification corresponds to the embodiment of the method according to the invention where in step E) the second adhesive layer is contacted with a mixture of the polyurethane (meth)acrylate compound and a solution of the polyurethane polymer selected in step A).

Hence, the layer of Carbothane® 125 was contacted with a 1:1 mixture of the 10 weight-% solution of Carbothane® 125 in chloroform (including UV starters, as well) and a 3.5 weight-% solution of Carbothane® 3585 in chloroform and an irradiation with UV light took place. The protocol is given below:
- degreasing of the nitinol stent with isopropanol and blasting with corundum
- PVD of SiO₂ layer
- Reaction of the PVD layer with the silane MEMO
- dip coating with the UV adhesive Loctite® 3301 and UV curing
- first dip coating with a solution of 10 weight-% of Carbothane® 125 in chloroform with 4 weight-% of benzophenone and 2 weight-% of hydroxycyclohexylphenyl ketone (based on the weight of Carbothane® 125); 9 minutes UV curing while rotating horizontally (ca. 250 rpm)
- second dip coating with a solution of 10 weight-% of Carbothane® 125 in chloroform with 4 weight-% of benzophenone and 2 weight-% of hydroxycyclohexylphenyl ketone (based on the weight of Carbothane® 125); 9 minutes UV curing while rotating horizontally (ca. 250 rpm)
- dip coating with a 1:1 mixture of the 10 weight-% solution of Carbothane® 125 in chloroform (including UV starters, see above) and a 3.5 weight-% solution of Carbothane® 3585 in chloroform; 9 minutes UV curing while rotating horizontally (ca. 250 rpm)
- dip coating with a 3.5 weight-% solution of Carbothane® 3585 in chloroform followed by rotating horizontally (ca. 250 rpm)

After this procedure the coated areas of the stent gave a milky appearance as opposed to the glossy surface obtained in example 4. Following a waiting period of 24 hours in order to allow the solvent to evaporate, a thermoformed PCU tube (Carbothane® 3585) in ventricular shape was bonded with the aid of a 10 weight-% solution of this PCU in chloroform to the exterior of the stent. Evaporation of the solvent gave the finished product. Testing of the product revealed no defects after ca. 10 million cycles. The pressure was then increased up to about 200 mm Hg. This led to a breaking of the stent itself. The bond between the PCU tube and the stent was still intact.

### Example 6: pulmonary stent

A bronchial stent made of nitinol material was treated as described in example 4 with the difference that the stent was only dipped once into the Carbothane® 125 solution before UV irradiation. Instead of bonding a thermoformed PCU material using a PCU solution, a fleece formed was by spraying a 7.5 weight-% solution of Carbothane® 3575 in chloroform directly onto the treated surface of the stent and allowing the solvent to evaporate.

Specimens thus obtained were subjected to crimping tests where they were crimped from a diameter of ca. 15 mm to a diameter of ca. 7.5 mm and relaxed for a total of 5 repetitions. No damage was observed, especially no damage to the bond between the fleece and the stent substrate.

Furthermore, in vivo experiments showed no signs of fleece separation after six months in the bronchial system of sheep.

### Example 7: heart valve stent (including crimping)

Samples prepared according to the procedure of example 5 having an external diameter of ca. 23 mm were cooled in ice water (to facilitate crimping) and crimped using an iris aperture type-mechanism to an external diameter of ca. 15 mm. The crimped stents were inserted into a tubular silicone rubber compartment and immersed in warm water, thereby expanding to their original diameter. Two compartment sizes were used: a regular size and an oversized version.

The durability of several specimens thus obtained was also tested in a continuous heart valve testing facility at 37 °C in an aqueous medium under dynamic load (600 beats per minute, 100 mm/150 mm Hg pressure). If a failure has occurred, the mode of failure was a fracture of the nitinol stent itself. The bond between the PCU material and the stent substrate remained intact. Results are summarized in the table below:

| **Sample's compartment size** | **Trial 1 - 100 mm Hg** | **Trial 2 - 150 mm Hg** |
|---|---|---|
| Standard | Survived > 10⁷ cycles | Survived > 10⁶ cycles |
| Oversized | Survived > 10⁷ cycles | Broken at < 72000 cycles |
| Standard | Survived > 10⁷ cycles | Survived > 10⁶ cycles |
| Oversized | Survived > 10⁷ cycles | Broken at < 360000 cycles |

It should be noted that an alternative adhesive, the epoxy adhesive Loctite® M31CL, was briefly considered for use in the manufacture of stents. However, it became apparent that the elasticity of this adhesive would be far too low to remain intact in crimping procedures. Furthermore, the miscibility of the epoxy adhesive in PCU solutions was disappointing.

## Claims

1. A method for bonding a polyurethane polymer to a substrate (100), the method comprising the steps of:
A) selecting a polyurethane polymer to be bonded to the substrate (100),
the polyurethane polymer being obtained by the reaction of a reaction mixture comprising a first isocyanate component and a first isocyanate-reactive component,
the first isocyanate-reactive component comprising a first polyol,
the first polyol being a member of a first class of polyols selected from polyether polyols, polyester polyols, polyetherester polyols, polycarbonate polyols, polyether carbonate polyols and polyacrylate polyols;
B) depositing an SiO₂ layer (200) onto the substrate (100);
C) contacting the SiO₂ layer (200) with a hydroxysilane compound comprising a carbon-carbon double bond and/or with an alkoxysilane compound comprising a carbon-carbon double bond
and subjecting to conditions suitable to at least partially effect a condensation reaction between Si-OH groups of the SiO₂ layer (200) and the hydroxysilane compound comprising a carbon-carbon double bond and/or the alkoxysilane compound comprising a carbon-carbon double bond,
thereby forming a first adhesive layer (300) bonded to the SiO₂ layer (200);
D) contacting the first adhesive layer (300) with a urethane (meth)acrylate compound
and subjecting to conditions suitable to at least partially effect a reaction between the carbon- carbon double bonds present in the first adhesive layer (300) and the urethane (meth)acrylate compound,
thereby forming a second adhesive layer (400) bonded to the first adhesive layer (300);
E) contacting the second adhesive layer (400) with a polyurethane (meth)acrylate compound,
the polyurethane (meth)acrylate compound being obtained by the reaction of a reaction mixture comprising a second isocyanate component and second isocyanate-reactive component,
the second isocyanate-reactive component comprising a second polyol,
the second polyol being a member of the first class of polyols as mentioned in step A),
the polyurethane (meth)acrylate compound furthermore comprising (meth)acrylate groups and subjecting to conditions suitable to at least partially effect a reaction between the (meth)acrylate groups and/or carbon radicals present in the second adhesive layer (400) and the polyurethane (meth)acrylate compound,
thereby forming a third adhesive layer (500) bonded to the second adhesive layer (400);
F) contacting the third adhesive layer (500) with the polyurethane polymer selected in step A), the polyurethane polymer being in a molten and/or dissolved state;
G) solidifying the polyurethane polymer of step F) which is in contact with the third adhesive layer by
cooling the polyurethane polymer below its melting point in the case that it has been employed in a molten state in step F);
and/or
evaporating the solvent in which the polyurethane polymer has been dissolved in the case that it has been employed in a dissolved state in step F);
thereby obtaining a polyurethane polymer layer (600);
thereby obtaining an article comprising a polyurethane polymer bonded to a substrate (100).

2. The method according to claim 1, wherein the material of the substrate (100) comprises nitinol, aluminium, steel and/or titanium.

3. The method according to claim 1 or 2, wherein the first polyol of the polyurethane polymer of step A) is a polycarbonate polyol.

4. The method according to one of claims 1 to 3, wherein in step B) the SiO₂ layer (200) is deposited by physical vapour deposition.

5. The method according to one of claims 1 to 4, wherein in step C) contacting the SiO₂ layer (200) is undertaken with an alkoxysilane compound comprising a (meth)acrylate group.

6. The method according to one of claims 1 to 5, wherein in step D) the conditions are irradiating with ultraviolet light in the presence of a UV initiator at an intensity of ≥ 5 mW/cm² to ≤ 50 mW/cm² for a duration of ≥ 1 minute to ≤ 10 minutes.

7. The method according to one of claims 1 to 6, wherein in step E) the conditions are irradiating with ultraviolet light in the presence of a UV initiator at an intensity of ≥ 5 mW/cm² to ≤ 100 mW/cm² for a duration of ≥ 1 minute to ≤ 10 minutes.

8. The method according to one of claims 1 to 7, wherein in step F) the third adhesive layer (500) is contacted with a solution of the polyurethane polymer selected in step A) and with solid polyurethane selected in step A).

9. The method according to one of claims 1 to 7, wherein in step F) the third adhesive layer (500) is contacted with a solution of the polyurethane polymer selected in step A) by spraying, through a nozzle moving relative to the third adhesive layer, a solution of the polyurethane polymer selected in step A) onto the third adhesive layer (500).

10. The method according to one of claims 1 to 9, wherein in step E) the second adhesive layer (400) is contacted with a mixture of the polyurethane (meth)acrylate compound and a solution of the polyurethane polymer selected in step A).

11. An article comprising a polyurethane polymer layer (600) bonded to a substrate (100), **characterized in that** the polyurethane polymer layer (600) is bonded to the substrate by an adhesive layer assembly comprising:
an SiO₂ layer (200) deposited onto the substrate (100);
a first adhesive layer (300) bonded to the SiO₂ layer (200);
a second adhesive layer (400) bonded to the first adhesive layer (300);
a third adhesive layer (500) bonded to the second adhesive layer (400) and bonded to the polyurethane;
the adhesive layer assembly being obtainable by a method according to one of claims 1 to 10.

12. The article according to claim 11, wherein the polyurethane polymer of the polyurethane polymer layer (600) is obtained by the reaction of a reaction mixture comprising a first isocyanate component and a first isocyanate-reactive component, the first isocyanate-reactive component comprising a first polyol, the first polyol being a member of the class of polycarbonate polyols.

13. The article according to claim 11 or 12, wherein the polyurethane layer (600) bridges opposing sections of the substrate (100).

14. The article according to one of claims 11 to 13, wherein the polyurethane layer (600) is disposed as a fleece.

15. The article according to one of claims 11 to 14, wherein the article is a biomedical implant.

## Patentansprüche

1. Verfahren zum Binden eines Polyurethanpolymers an ein Substrat (100), bei dem man:
A) ein an das Substrat (100) zu bindendes Polyurethanpolymer auswählt,
wobei das Polyurethanpolymer durch die Reaktion einer Reaktionsmischung, die eine erste Isocyanatkomponente und eine zweite Isocyanat-reaktive Komponente umfasst, erhalten wird,
wobei die erste Isocyanat-reaktive Komponente ein erstes Polyol umfasst,
wobei das erste Polyol ein Mitglied einer ersten Klasse von Polyolen ausgewählt aus Polyetherpolyolen, Polyesterpolyolen, Polyetheresterpolyolen, Polycarbonatpolyolen, Polyethercarbonatpolyolen und Polyacrylatpolyolen ist;
B) auf dem Substrat (100) eine SiO₂-Schicht (200) abscheidet;
C) die SiO₂-Schicht (200) mit einer Hydroxysilan-Verbindung mit einer Kohlenstoff-Kohlenstoff-Doppelbindung und/oder mit einer Alkoxysilan-Verbindung mit einer Kohlenstoff-Kohlenstoff-Doppelbindung in Kontakt bringt
und Bedingungen unterwirft, die zur zumindest teilweisen Bewirkung einer Kondensationsreaktion zwischen Si-OH-Gruppen der SiO₂-Schicht (200) und der Hydroxysilan-Verbindung mit einer Kohlenstoff-Kohlenstoff-Doppelbindung und/oder der Alkoxysilan-Verbindung mit einer Kohlenstoff-Kohlenstoff-Doppelbindung geeignet sind, wodurch eine an die SiO₂-Schicht (200) gebundene erste Haftschicht (300) gebildet wird;
D) die erste Haftschicht (300) mit einer Urethan-(meth)acrylat-Verbindung in Kontakt bringt und Bedingungen unterwirft, die zur zumindest teilweisen Bewirkung einer Reaktion zwischen den in der ersten Haftschicht (300) vorliegenden Kohlenstoff-Kohlenstoff-Doppelbindungen und der Urethan(meth)acrylat-Verbindung geeignet sind,
wodurch auf der ersten Haftschicht (300) eine zweite Haftschicht (400) gebildet wird;
E) die zweite Haftschicht (400) mit einer Polyurethan(meth)acrylat-Verbindung in Kontakt bringt,
wobei die Polyurethan(meth)acrylat-Verbindung durch die Reaktion einer Reaktionsmischung, die eine zweite Isocyanatkomponente und eine zweite Isocyanat-reaktive Komponente umfasst, erhalten wird,
wobei die zweite Isocyanat-reaktive Komponente ein zweites Polyol umfasst,
wobei das zweite Polyol ein Mitglied der ersten Klasse von Polyolen gemäß den Angaben in Schritt A) ist;
wobei die Polyurethan(meth)acrylat-Verbindung ferner (Meth)acrylatgruppen umfasst,
und Bedingungen unterwirft, die zur zumindest teilweisen Bewirkung einer Reaktion zwischen den in der zweiten Haftschicht (400) vorliegenden(Meth)acrylatgruppen und/oder Kohlenstoffresten und der Polyurethan(meth)acrylat-Verbindung geeignet sind,
wodurch auf der zweiten Haftschicht (400) eine dritte Haftschicht (500) gebildet wird;
F) die dritte Haftschicht (500) mit dem in Schritt (A) ausgewählten Polyurethanpolymer in Kontakt bringt,
wobei das Polyurethanpolymer in schmelzflüssigem und/oder gelöstem Zustand vorliegt;
D) Verfestigen des Polyurethanpolymers aus Schritt F), das mit der dritten Haftschicht in Kontakt steht, durch
Abkühlen des Polyurethanpolymers unter seinen Schmelzpunkt in dem Fall, dass es in Schritt F) in schmelzflüssigem Zustand eingesetzt wurde;
und/oder
Verdampfen des Lösungsmittels, in dem das Polyurethanpolymer gelöst wurde, in dem Fall, dass es in Schritt F) in gelöstem Zustand eingesetzt wurde;
wodurch eine Polyurethanpolymerschicht (600) erhalten wird;
wodurch ein Artikel erhalten wird, der ein an ein Substrat (100) gebundenes Polyurethanpolymer umfasst.

2. Verfahren nach Anspruch 1, wobei das Material des Substrats (100) Nitinol, Aluminium, Stahl und/oder Titan umfasst.

3. Verfahren nach Anspruch 1 oder 2, wobei es sich bei dem ersten Polyol des Polyurethanpolymers von Schritt A) um ein Polycarbonatpolyol handelt.

4. Verfahren nach einem der Ansprüche 1 bis 3, wobei in Schritt B) die Abscheidung der SiO₂-Schicht (200) durch physikalische Dampfabscheidung erfolgt.

5. Verfahren nach einem der Ansprüche 1 bis 4, wobei in Schritt C) das Inkontaktbringen der SiO₂-Schicht (200) mit einer Alkoxysilan-Verbindung mit einer (Meth)acrylatgruppe durchgeführt wird.

6. Verfahren nach einem der Ansprüche 1 bis 5, wobei es sich in Schritt D) bei den Bedingungen um Bestrahlung mit ultraviolettem Licht in Gegenwart eines UV-Initiators mit einer Intensität von ≥ 5 mW/cm² bis ≤ 50 mW/cm² für eine Dauer von ≥ 1 Minute bis ≤ 10 Minuten handelt.

7. Verfahren nach einem der Ansprüche 1 bis 6, wobei es sich in Schritt E) bei den Bedingungen um Bestrahlung mit ultraviolettem Licht in Gegenwart eines UV-Initiators mit einer Intensität von ≥ 5 mW/cm² bis ≤ 100 mW/cm² für eine Dauer von ≥ 1 Minute bis ≤ 10 Minuten handelt.

8. Verfahren nach einem der Ansprüche 1 bis 7, wobei in Schritt F) die dritte Haftschicht (500) mit einer Lösung des in Schritt A) ausgewählten Polyurethanpolymers und mit in Schritt A) ausgewähltem festem Polyurethan in Kontakt gebracht wird.

9. Verfahren nach einem der Ansprüche 1 bis 7, wobei in Schritt F) die dritte Haftschicht (500) mit einer Lösung des in Schritt A) ausgewählten Polyurethanpolymers in Kontakt gebracht wird, indem eine Lösung des in Schritt A) ausgewählten Polyurethanpolymers auf die dritte Haftschicht (500) durch eine sich relativ zu der dritten Haftschicht bewegende Düse aufgesprüht wird.

10. Verfahren nach einem der Ansprüche 1 bis 9, wobei in Schritt E) die zweite Haftschicht (400) mit einer Mischung der Polyurethan(meth)acrylat-Verbindung und einer Lösung des in Schritt A) ausgewählten Polyurethanpolymers in Kontakt gebracht wird.

11. Artikel, umfassend eine an ein Substrat (100) gebundene Polyurethanpolymerschicht (600), **dadurch gekennzeichnet, dass** die Polyurethanpolymerschicht (600) über eine Haftschichtanordnung, umfassend:
eine auf dem Substrat (100) abgeschiedene SiO₂-Schicht (200);
eine an die SiO₂-Schicht (200) gebundene erste Haftschicht (300);
eine an die erste Haftschicht (300) gebundene zweite Haftschicht (400);
eine an die zweite Haftschicht (400) und an das Polyurethan gebundene dritte Haftschicht (500);
an das Substrat gebunden ist;
wobei die Haftschichtanordnung durch ein Verfahren nach einem der Ansprüche 1 bis 10 erhältlich ist.

12. Artikel nach Anspruch 11, wobei das Polyurethanpolymer der Polyurethanpolymerschicht (600) durch die Reaktion einer Reaktionsmischung, die eine erste Polyisocyanatkomponente und eine erste Isocyanat-reaktive Komponente umfasst, erhalten wird, wobei die erste Isocyanat-reaktive Komponente ein erstes Polyol umfasst, wobei es sich bei dem ersten Polyol um ein Mitglied der Klasse der Polycarbonatpolyole handelt.

13. Artikel nach Anspruch 11 oder 12, wobei die Polyurethanschicht (600) gegenüberliegende Abschnitte des Substrats (100) überbrückt.

14. Artikel nach einem der Ansprüche 11 bis 13, wobei die Polyurethanschicht (600) als Vlies ausgelegt ist.

15. Artikel nach einem der Ansprüche 11 bis 14, wobei es sich bei dem Artikel um ein biomedizinisches Implantat handelt.

## Revendications

1. Procédé de liaison d'un polymère de polyuréthane à un substrat (100), le procédé comprenant les étapes de :
A) sélection d'un polymère de polyuréthane devant être lié au substrat (100),
le polymère de polyuréthane étant obtenu par la réaction d'un mélange de réaction comprenant un premier composant d'isocyanate et un premier composant réactif avec l'isocyanate,
le premier composant réactif avec l'isocyanate comprenant un premier polyol,
le premier polyol étant un membre d'une première classe de polyols choisie parmi les polyéther polyols, les polyester polyols, les polyétherester polyols, les polycarbonate polyols, les polyéther carbonate polyols et les polyacrylate polyols ;
B) dépôt d'une couche de SiO₂ (200) sur le substrat (100) ;
C) mise en contact de la couche de SiO₂ (200) avec un composé d'hydroxysilane comprenant une double liaison carbone-carbone et/ou avec un composé d'alcoxysilane comprenant une double liaison carbone-carbone
et soumission à des conditions adaptées pour effectuer au moins partiellement une réaction de condensation entre les groupes Si-OH de la couche de SiO₂ (200) et le composé d'hydroxysilane comprenant une double liaison carbone-carbone et/ou le composé d'alcoxysilane comprenant une double liaison carbone-carbone,
de façon à former une première couche adhésive (300) liée à la couche de SiO₂ (200) ;
D) mise en contact de la première couche adhésive (300) avec un composé de (méth)acrylate d'uréthane et soumission à des conditions adaptées pour effectuer au moins partiellement une réaction entre les doubles liaisons carbone-carbone présentes dans la première couche adhésive (300) et le composé de (méth)acrylate d'uréthane,
de façon à former une deuxième couche adhésive (400) liée à la première couche adhésive (300) ;
E) mise en contact de la deuxième couche adhésive (400) avec un composé de (méth)acrylate de polyuréthane,
le composé de (méth)acrylate de polyuréthane étant obtenu par la réaction d'un mélange de réaction comprenant un deuxième composant d'isocyanate et un deuxième composant réactif avec l'isocyanate,
le deuxième composant réactif avec l'isocyanate comprenant un deuxième polyol,
le deuxième polyol étant un membre de la première classe de polyols telle que mentionnée dans l'étape A),
le composé de (méth)acrylate de polyuréthane comprenant en outre des groupes (méth)acrylate et soumission à des conditions adaptées pour effectuer au moins partiellement une réaction entre les groupes (méth)acrylate et/ou les radicaux carbonés présents dans la deuxième couche adhésive (400) et le composé de (méth)acrylate de polyuréthane,
de façon à former une troisième couche adhésive (500) liée à la deuxième couche adhésive (400) ;
F) mise en contact de la troisième couche adhésive (500) avec le polymère de polyuréthane sélectionné dans l'étape A),
le polymère de polyuréthane étant dans un état fondu et/ou dissous ;
G) solidification du polymère de polyuréthane de l'étape F) qui est en contact avec la troisième couche adhésive par
refroidissement du polymère de polyuréthane au-dessous de son point de fusion dans le cas où celui-ci a été utilisé dans un état fondu dans l'étape F) ; et/ou
évaporation du solvant dans lequel le polymère de polyuréthane a été dissous dans le cas où celui-ci a été utilisé dans un état dissous dans l'étape F) ; de façon à obtenir une couche de polymère de polyuréthane (600) ;
de façon à obtenir un article comprenant un polymère de polyuréthane lié à un substrat (100).

2. Procédé selon la revendication 1, dans lequel le matériau du substrat (100) comprend le nitinol, l'aluminium, l'acier et/ou le titane.

3. Procédé selon la revendication 1 ou 2, dans lequel le premier polyol du polymère de polyuréthane de l'étape A) est un polycarbonate polyol.

4. Procédé selon l'une des revendications 1 à 3, dans lequel, dans l'étape B), la couche de SiO₂ (200) est déposée par dépôt physique en phase vapeur.

5. Procédé selon l'une des revendications 1 à 4, dans lequel, dans l'étape C), la mise en contact de la couche de SiO₂ (200) est conduite avec un composé d'alcoxysilane comprenant un groupe (méth)acrylate.

6. Procédé selon l'une des revendications 1 à 5, dans lequel, dans l'étape D) les conditions sont une irradiation avec une lumière ultraviolette en présence d'un amorceur UV à une intensité de ≥ 5 mW/cm² à ≤ 50 mW/cm² pendant une durée de ≥ 1 minute à ≤ 10 minutes.

7. Procédé selon l'une des revendications 1 à 6, dans lequel, dans l'étape E) les conditions sont une irradiation avec une lumière ultraviolette en présence d'un amorceur UV à une intensité de ≥ 5 mW/cm² à ≤ 100 mW/cm² pendant une durée de ≥ 1 minute à ≤ 10 minutes.

8. Procédé selon l'une des revendications 1 à 7, dans lequel, dans l'étape F) la troisième couche adhésive (500) est mise en contact avec une solution du polymère de polyuréthane sélectionné dans l'étape A) et avec le polyuréthane sélectionné dans l'étape A) solide.

9. Procédé selon l'une des revendications 1 à 7, dans lequel, dans l'étape F), la troisième couche adhésive (500) est mise en contact avec une solution du polymère de polyuréthane sélectionné dans l'étape A) par pulvérisation, par l'intermédiaire d'une buse se déplaçant par rapport à la troisième couche adhésive, d'une solution du polymère de polyuréthane sélectionné dans l'étape A) sur la troisième couche adhésive (500).

10. Procédé selon l'une des revendications 1 à 9, dans lequel, dans l'étape E), la deuxième couche adhésive (400) est mise en contact avec un mélange du composé de (méth)acrylate de polyuréthane et d'une solution du polymère de polyuréthane sélectionné dans l'étape A).

11. Article comprenant une couche de polymère de polyuréthane (600) liée à un substrat (100), **caractérisé en ce que** la couche de polymère de polyuréthane (600) est liée au substrat par un assemblage de couches adhésives comprenant :
une couche de SiO₂ (200) déposée sur le substrat (100) ;
une première couche adhésive (300) liée à la couche de SiO₂ (200) ;
une deuxième couche adhésive (400) liée à la première couche adhésive (300) ;
une troisième couche adhésive (500) liée à la deuxième couche adhésive (400) et liée au polyuréthane ;
l'assemblage de couches adhésives pouvant être obtenu par un procédé selon l'une des revendications 1 à 10.

12. Article selon la revendication 11, dans lequel le polymère de polyuréthane de la couche de polymère de polyuréthane (600) est obtenu par la réaction d'un mélange de réaction comprenant un premier composant d'isocyanate et un premier composant réactif avec l'isocyanate, le premier composant réactif avec l'isocyanate comprenant un premier polyol, le premier polyol étant un membre de la classe des polycarbonate polyols.

13. Article selon la revendication 11 ou 12, dans lequel la couche de polyuréthane (600) ponte des sections opposées du substrat (100).

14. Article selon l'une des revendications 11 à 13, dans lequel la couche de polyuréthane (600) est disposée sous la forme d'un voile.

15. Article selon l'une des revendications 11 à 14, l'article étant un implant biomédical.
